(19) Europäisches Patentamt
European Patent Office
Office européen des brevets

(11) Veröffentlichungsnummer: **0 321 854**
**A1**

# (12) EUROPÄISCHE PATENTANMELDUNG

(21) Anmeldenummer: **88120955.5**

(51) Int. Cl.⁴: **C11D 11/04 , C07C 141/04**

(22) Anmeldetag: **15.12.88**

(30) Priorität: **23.12.87 DE 3743823**

(43) Veröffentlichungstag der Anmeldung: **28.06.89 Patentblatt 89/26**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Billenstein, Siegfried, Dr.**
**Samerbergweg 8**
**D-8269 Burgkirchen(DE)**
Erfinder: **Ehrl, Winfried, Dr.**
**Stethaimerstrasse 4**
**D-8265 Neuötting(DE)**

## (54) Verfahren zur Herstellung eines Ethersulfat enthaltenden Tensidgemisches mit niedrigem Dioxangehalt.

(57) Nach dem neuen Verfahren wird eine Mischung von 99 bis 5 Mol-% mindestens eines Alkanoloxethylates und 1 bis 95 Mol-% von mindestens einem Carbonsäureamidoxethylat in flüssigem Zustand bei 20 bis 120 °C mit 0,95 bis 1 mol Chlorsulfonsäure oder Schwefeltrioxid je 1 mol, der in den Oxethylaten enthaltenen $-CH_2CH_2OH$-Gruppen umgesetzt und das Reaktionsprodukt anschließend mit einer wäßrigen Lösung oder Aufschlämmung eines Alkali-, Erdalkali- oder Ammoniumhydroxids neutralisiert. Es werden so in Wasser gelöste Ethersulfat enthaltende Tensidgemische mit niedrigem Gehalt an 1,4-Dioxan erhalten, die entweder direkt oder nach kostengünstiger Entfernung der geringen Mengen von Rest-1,4-Dioxan als Körperpflegemittel, Geschirrspülmittel oder Waschmittel eingesetzt werden können.

EP 0 321 854 A1

## Verfahren zur Herstellung eines Ethersulfat enthaltenden Tensidgemisches mit niedrigem Dioxangehalt

Die Erfindung betrifft ein Verfahren zur Herstellung eines Ethersulfat enthaltenden Tensidgemisches und ein solches Tensidgemisch mit niedrigem Dioxangehalt.

Ethersulfate werden technisch hergestellt durch Umsetzung von Alkanolen oder Alkylphenolen mit Alkylenoxid, meist Ethylenoxid, die Alkylenoxid-Anlagerungsprodukte werden anschließend mit Mitteln, wie Chlorsulfonsäure oder Schwefeltrioxid sulfatiert, wobei Schwefelsäurehalbester entstehen, die nachfolgend mit alkalischen Verbindungen, beispielsweise Alkalihydroxiden, in wäßriger Lösung neutralisiert werden. Bei der Sulfatierung entsteht unter anderem als Nebenprodukt 1,4-Dioxan in Mengen von 0,5 bis 0,03 Gew.-%, bezogen auf Ethersulfat.

Aus toxikologischen Untersuchungen von R.J. Kociba et al., Toxicology, Applied Pharmacology,Vol. 30 (1974), Seiten 275 bis 298, geht hervor, daß 1,4-Dioxan im Tierversuch, allerdings erst bei Anwendung sehr großer Mengen, gesundheitliche Schädigungen erzeugt. Obwohl aus diesen Versuchen keine direkte Gefahr bei der Verarbeitung und Anwendung von Ethersulfaten herleitbar ist, besteht doch seither bei Herstellern und Anwendern der Ethersulfate, vor allem bei der Anwendung im kosmetischen Bereich, ein erhebliches Interesse an Produkten, die einen möglichst weitgehend verminderten Gehalt an 1,4-Dioxan aufweisen, um jedes Risiko auszuschalten.

Zur Herabsetzung des 1,4-Dioxangehaltes in Ethersulfaten sind verschiedene Verfahren bekannt, die alle eine Entfernung des 1,4-Dioxans durch Wärme beziehungsweise Wasserdampfbehandlung des fertigen Ethersulfates bezwecken. So wird in der US-PS 4,285,881 ein Verfahren beschrieben, Ethersulfat/Dioxan-Mischungen mit dioxanfreiem Wasserdampf, bei Temperaturen von 25 bis 150 °C, in einer Abstreif-Vorrichtung in Kontakt zu bringen, wobei die Ethersulfat/Dioxan-Mischung vorteilhaft in dünner Schicht fließend angewendet wird. Nach DE 31 26 175-A1 werden Ethersulfate in konzentrierter 60 bis 80 gew.%iger wäßriger Lösung einer Wärmebehandlung bei 50 bis 130 °C unter vermindertem Druck unterworfen, um sie geruchfrei zu machen, wobei auch bei der Sulfatierung als Nebenprodukt gebildete cyclische Ether (1,4-Dioxan) entfernt werden. In DE 30 44 488-A1 ist ein ähnliches Verfahren beschrieben, mit dem direkt genannten Ziel, Ethersulfate mit erniedrigtem Gehalt an 1,4-Dioxan zu erzeugen. Diese Verfahren sind apparativ aufwendig und benötigen nicht unbeträchtliche Energiemengen. Ferner beseitigen sie nicht mögliche Gefahren, die bei der Sulfatierung und Neutralisation vom Gehalt der Reaktionsmischungen an 1,4-Dioxan ausgehen.

Es wurde nun ein Verfahren gefunden, das mit den üblichen Sulfatierungsmitteln Chlorsulfonsäure oder Schwefeltrioxid durchgeführt werden kann, wobei wesentlich geringere Mengen an 1,4-Dioxan bei der Herstellung des Ethersulfates entstehen, so daß ein nachträglicher Reinigungsaufwand entweder entfällt oder bedeutend vermindert werden kann. Das neue Verfahren beruht auf der überraschenden Feststellung, daß bereits vergleichsweise geringe Zusatzmengen von Carbonsäureamidoxethylaten zu den Alkanoloxethylaten die Neigung zur Bildung von 1,4-Dioxan bei der Sulfatierung wesentlich vermindern. So sind nach der üblichen Neutralisation Tensidgemische erhältlich, die bereits von der Herstellung her deutlich weniger 1,4-Dioxan enthalten, als die Gemische vergleichbarer Zusammensetzung aufweisen, bei denen die Einzelkomponenten vor der Mischung getrennt sulfatiert und neutralisiert wurden.

Das neue Verfahren zur Herstellung eines Ethersulfat enthaltenden Tensidgemisches mit niedrigem Dioxangehalt, ist dadurch gekennzeichnet, daß eine Mischung, bestehend aus 99 bis 5 Mol-%, bezogen auf die Mischung, von mindestens einer Verbindung der Formel

$$R\text{-}O\text{-}(CH_2CH_2O)_aH \quad (I)$$

worin bedeuten

R einen Alkylrest mit 8 bis 18 C-Atomen oder einen Alkylphenylrest, der mit 1 bis 3 Alkylresten substituiert ist, wobei die Gesamtzahl der C-Atome in den Alkylresten 4 bis 12 beträgt,

a eine Zahl von 1 bis 10 und

1 bis 95 Mol-%, bezogen auf die Mischung, von mindestens einer Verbindung der Formel

$$R^1\text{-CO-N}\begin{matrix}\diagup(CH_2CH_2O)_bH\\ \diagdown(CH_2CH_2O)_cH\end{matrix} \qquad (II),$$

worin bedeuten

$R^1$ einen Alkylrest mit 1 bis 17 C-Atomen

b eine Zahl von 1 bis 20 und

c Null oder eine Zahl von 1 bis 20

in flüssigem Zustand bei 20 bis 120 °C mit 0,95 bis 1 mol Chlorsulfonsäure oder $SO_3$ je 1 mol der in den Verbindungen der Formeln (I) und (II) enthaltenen $-CH_2CH_2OH$-Gruppen umgesetzt werden und das Reaktionsprodukt anschließend mit einer wäßrigen Lösung oder Aufschlämmung einer Verbindung der Formel MOH, worin bedeutet M = Na; K; Li; 1/2 Mg; 1/2 Ca; 1/2 Ba; oder

$$\begin{matrix} & R^3 & \\ & | & \\ R^2- & N & -R^4 \\ & | & \\ & R^5 & \end{matrix},$$

worin $R^2$ bis $R^5$ gleich oder verschieden sein können und jedes für sich bedeutet: H; Alkyl mit 1 bis 4 C-Atomen oder $-CH_2CH_2OH$, neutralisiert wird.

Für das erfindungsgemäße Verfahren sind Umsetzungsprodukte von Ethylenoxid mit Alkanolen geeignet, deren Alkylrest geradkettig oder verzweigt sein kann und auch in der Kohlenstoffkette Doppelbindungen enthalten kann. Er soll 8 bis 18 C-Atome enthalten. Wegen ihrer guten Eigenschaften sind solche Alkanolethylenoxid-Umsetzungsprodukte bevorzugt, die im Alkylrest 10 bis 16 C-Atome enthalten. Die Anzahl der an das Alkanol angelagerten Ethylenoxid-Einheiten soll zwischen 1 und 10 liegen, wobei solche Alkanol-Umsetzungsprodukte bevorzugt sind, bei denen 2 bis 4 Ethylenoxid-Einheiten angelagert sind.

Ebenso für das erfindungsgemäße Verfahren sind geeignet Ethylenoxid-Anlagerungsprodukte an Alkylphenole, die mit 1 bis 3 Alkylresten substituiert sind, wobei die Gesamtzahl der Kohlenstoffatome in dem Alkylrest beziehungsweise den Alkylresten 4 bis 12 betragen soll. Einer oder mehrere dieser Alkylreste können verzweigt sein. Die Alkylphenole sollen 1 bis 10, vorzugsweise 4 bis 8,Ethylenoxid-Einheiten angelagert enthalten.

Es ist nicht erforderlich, für das erfindungsgemäße Verfahren nur eine Verbindung der Formel (I) einzusetzen. Ebensogut können Gemische verschiedener solcher Verbindungen verwendet werden, in denen verschiedene Alkylreste oder auch Alkylphenylreste vorliegen und/oder in denen auch verschiedene Anzahlen von Ethylenoxid-Einheiten an das Alkanol beziehungsweise Alkylphenol angelagert sind. - Die Herstellung der Ethylenoxid-Anlagerungsprodukte an das Alkanol beziehungsweise Alkylphenol ist dem Fachmann bekannt, beispielsweise aus Ullmann's Encyklopädie der technischen Chemie, 4. Auflage (1982), Band 22, Seiten 489 bis 493.

Als zweite Mischungskomponente werden für das erfindungsgemäße Verfahren Ethylenoxid-Anlagerungsprodukte an Carbonsäureamide verwendet, die sich von Carbonsäuren ableiten, deren Alkylrest 1 bis 17 C-Atome enthält und geradkettig oder verzweigt sein kann sowie auch Doppelbindungen in der Kohlenstoffkette enthalten kann. Wegen der guten Tensid-Eigenschaften sind solche Ethylenoxid-Anlagerungsprodukte an Carbonsäureamide bevorzugt, die im Alkylrest der Carbonsäure 7 bis 17 C-Atome enthalten. Die Anlagerung des Ethylenoxids kann sowohl nur an einem wie auch an beiden am Stickstoff gebundenen Wasserstoffatomen des Carbonsäureamids erfolgen. Solche Verbindungen werden üblicherweise hergestellt, indem zunächst das Carbonsäuremethylester mit Mono- oder Diethanolamin umgesetzt wird,und dieses Umsetzungsprodukt anschließend mit Ethylenoxid weiter oxethyliert wird (siehe beispielsweise Ullmann's Encyklopädie der technischen Chemie, loc. cit., Seite 493 sowie auch Kurt Lindner "Tenside -Textilhilfsmittel - Waschrohstoffe", Wissenschaftliche Verlagsgesellschaft mbH, Stuttgart, 1964, Band 1, Seiten 904 bis 907 und 912 bis 914). An jedes am Stickstoff gebundene Wasserstoffatom des Carbonsäureamides können 1 bis 20 Ethylenoxid-Einheiten angelagert sein. Wegen ihrer guten Zugänglichkeit und günstigen Wirkung sind solche Verbindungen bevorzugt, bei denen an nur einem am Stickstoff des Carbonsäureamids gebundenen Wasserstoffatom 1 bis 6 Ethylenoxid-Einheiten angelagert sind.

Wie oben bereits bei den Alkanoloxethylaten ausgeführt, ist es auch bei den Ethylenoxid-Anlagerungsprodukten an die Carbonsäureamide nicht erforderlich nur eine Verbindung einer bestimmten Formel einzusetzen, sondern es können oft sogar mit Vorteil Mischungen von Verbindungen der Formel (II) verwendet werden, die sich in der Anzahl der C-Atome im Alkylrest der Carbonsäureamidgruppe und/oder in der Zahl der an 1 oder 2 am Stickstoff gebundener Wasserstoffatome angelagerten Ethylenoxid-Einheiten unterscheiden.

Erfindungsgemäß wird eine Mischung von 99 bis 5 Mol-%, bezogen auf die Mischung, von mindestens einem Alkanoloxethylat oder Alkylphenoloxethylat der Formel (I) und 1 bis 95 Mol-%, bezogen auf die Mischung, von mindestens einem Carbonsäureamidoxethylat der Formel (II) in flüssigem Zustand mit Chlorsulfonsäure oder $SO_3$ umgesetzt. Unter einem Mol-%-Gehalt der Mischung an Verbindungen der Formel (II) wird die erfindungsgemäße 1,4-Dioxan vermindernde Wirkung des Zusatzes nur noch in geringem Maße festgestellt, über 95 Mol-%-Gehalt der Mischung an Verbindungen der Formel (II) ist zwar deren 1,4-Dioxan vermindernde Wirkung weiterhin vorhanden, wirkt sich aber wegen Anwesenheit einer vergleichsweise geringen Menge von Verbindungen der Formel (I) nicht mehr in technisch interessantem Maße aus. Vorzugsweise wird eine Mischung, die 97 bis 30 Mol-%, bezogen auf die Mischung, von mindestens einer Verbindung der Formel (I) und 3 bis 70 Mol-%, bezogen auf die Mischung, von mindestens einer Verbindung der Formel (II),enthält, mit Chlorsulfonsäure oder $SO_3$ umgesetzt.

Die Temperatur bei der Umsetzung mit Chlorsulfonsäure oder $SO_3$ beträgt zweckmäßig 20 bis 120 °C. Oberhalb 120 °C treten im allgemeinen zu viele Nebenreaktionen auf, die unter anderem zu einer Verfärbung der Produkte führen. Umsetzungstemperaturen unter 20 °C sind prinzipiell möglich, jedoch im allgemeinen nicht vorteilhaft, da die Viskosität der umzusetzenden Oxethylatgemische unnötig hoch wird und, sofern die Sulfatierung mit $SO_3$ in der Gasphase angewendet wird, auch Schwierigkeiten durch Auskristallisieren des $SO_3$ entstehen können. Vorzugsweise wird eine Umsetzungstemperatur von 30 bis 80 °C gewählt.

Grundsätzlich können für die erfindungsgemäße Reaktion auch Oxethylate von Dicarbonsäurediamiden eingesetzt werden, bei denen an je ein H-Atom, das an jedem der beiden Stickstoffatome gebunden ist, Ethylenoxid angelagert ist, oder bei denen an alle vier an den beiden Stickstoffatomen gebundenen Wasserstoffatomen Ethylenoxid angelagert wurde. Diese Dicarbonsäuren können geradkettige oder gegebenenfalls verzweigte Alkylenreste mit 1 bis 18 C-Atomen enthalten. Sie können ferner 2 bis 20 Ethylenoxid-Einheiten an die an den Stickstoffatomen gebundenen Wasserstoffatome angelagert aufweisen. Allerdings sind die Dicarbonsäurediamidoxethylate für das erfindungsgemäße Verfahren weniger interessant, da sie im allgemeinen einen höheren Schmelzpunkt als die Verbindungen der Formel (II) haben, was höhere Sulfatierungstemperaturen erforderlich macht und weil sie auch im allgemeinen schwerer beschaffbar sind.

Wie bereits erwähnt, wird die Umsetzung mit Chlorsulfonsäure oder mit $SO_3$ mit einer flüssigen Mischung der Verbindungen der Formeln (I) und (II) durchgeführt. Die Reaktionstemperatur wird zweckmäßig so gewählt, daß die Viskosität der flüssigen Mischung ein Rühren oder Herabrieselnlassen, beispielsweise in einem Fallfilmreaktor, ohne Schwierigkeiten ermöglicht. Zur Verminderung der Viskosität können Lösungsmittel, die weder mit den Oxethylaten noch mit Chlorsulfonsäure oder $SO_3$ reagieren, verwendet werden, beispielsweise halogenierte Kohlenwasserstoffe. In allgemeinen ist aber die Verwendung von Lösungsmitteln nicht erforderlich.

Die Reaktion der Mischung der Verbindungen der Formeln (I) und (II) mit Chlorsulfonsäure oder $SO_3$ erfolgt so, daß je 1 mol der in der Mischung enthaltenen $-CH_2CH_2OH$-Gruppen 0,95 bis 1 mol Chlorsulfonsäure oder $SO_3$ umgesetzt werden.

Beispielsweise wird die OH-Zahl der Mischung der Verbindungen der Formeln (I) und (II) ermittelt und die danach berechnete Menge Chlorsulfonsäure oder $SO_3$ unter Bewegung und Kühlen der Reaktionsmischung in einem so bemessenen Zeitraum zugesetzt, daß die Reaktionstemperatur nicht über den angestrebten Wert zwischen 20 und 120 °C ansteigt.

In einer bevorzugten Ausführungsform des erfindungsgemäßen Verfahrens wird die Mischung der Verbindungen der Formeln (I) und (II) mit einer Gasmischung, die neben nicht reagierenden Gas 0,5 bis 10 Vol.-%, vorzugsweise 1 bis 5 Vol.-%, $SO_3$ enthält, solange in Kontakt gebracht, bis je 1 mol der in den Verbindungen (I) und (II) enthaltenen $-CH_2CH_2OH$-Gruppen 0,95 bis 1 mol $SO_3$ von der Mischung der Verbindungen der Formeln (I) und (II) aufgenommen worden sind. Wann dies der Fall ist, kann beispielsweise durch Bestimmung der Säurezahl der Reaktionsmischung festgestellt werden.

Die Umsetzung mit Chlorsulfonsäure wird zweckmäßig bei normalem Atmosphärendruck durchgeführt, die Umsetzung mit $SO_3$, gegebenenfalls in Mischung mit nicht-reagierenden Gasen, kann zweckmäßig bei Drucken von 0,5 bis 100 kPa stattfinden, vorzugsweise wird auch hier normaler Atmosphärendruck angewendet.

Als nicht-reagierende Gase für die Mischung mit $SO_3$ sind beispielsweise geeignet: Luft, Stickstoff, Kohlendioxid oder Edelgase, beispielsweise Argon. Diese Gase sind möglichst weitgehend wasserdampffrei

anzuwenden.

Für die Herstellung des Gemisches aus nicht-reagierendem Gas und $SO_3$ sowie dessen Umsetzung mit der flüssigen Mischung der Verbindungen der Formeln (I) und (II) sind bekannte technische Einrichtungen und Apparate geeignet, wie sie beispielsweise in "Seifen - Öle - Fette - Wachse", 107. Jahrgang (1981), Seiten 47 bis 51, beschrieben sind.

Nachdem mit der Mischung der Verbindungen der Formeln (I) und (II) die weiter oben beschriebenen Mengen Chlorsulfonsäure beziehungsweise $SO_3$ umgesetzt worden sind, wird die Zufuhr der Sulfatierungsmittel unterbrochen, das Reaktionsgemisch gegebenenfalls gekühlt und unverzüglich unter Kühlung neutralisiert. Hierzu wird eine wäßrige Lösung mindestens einer Verbindung der Formel MOH verwendet, in der bedeuten: M = Na; K; Li; 1/2 Mg; 1/2 Ca; 1/2 Ba; oder

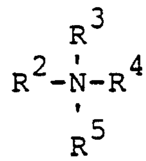 ,

worin $R^2$ bis $R^5$ gleich oder verschieden sein können und jedes für sich bedeutet: H; Alkyl mit 1 bis 4 C-Atomen oder $-CH_2CH_2OH$. 1/2 Mg bedeutet, daß zur Neutralisation die Verbindung $Mg(OH)_2$ eingesetzt wird, wobei jedoch nur 1/2 der molaren Menge dieser Verbindung im Vergleich beispielsweise zu einer Verbindung der Formel NaOH zur Neutralisation verbraucht werden. Analoge Bedeutungen haben 1/2 Ca und 1/2 Ba. Neutralisieren im Sinne der vorliegenden Erfindung heißt, daß die wäßrige Lösung des neutralisierten Produktes einen pH-Wert im Bereich von 6,5 bis 9 aufweist. Vorzugsweise erfolgt die Neutralisation mit einer wäßrigen Lösung mindestens einer Verbindung der Formel MOH, worin M = Na; K; 1/2 Mg; $NH_4$ oder $NH(CH_2CH_2OH)_3$ ist.

Der Gehalt der wäßrigen Lösung an der (oder den) Verbindung(en) der Formel MOH richtet sich nach dem angestrebten Wassergehalt des erfindungsgemäß hergestellten Tensidgemisches. Im allgemeinen werden Wassergehalte von 20 bis 70 Gew.-%, bezogen auf die wäßrige Lösung des Tensidgemisches, angewendet, höhere Wassergehalte sind ohne weiteres möglich, jedoch aus wirtschaftlichen Gründen (hohe Transportgewichte und Volumina) weniger interessant. Sofern in den anzuwendenden Wassermengen die Verbindung beziehungsweise die Verbindungen der Formel MOH nicht vollständig löslich sind, wird eine wäßrige Aufschlämmung angewendet, die diese Verbindung(en) teilweise in feinverteilter fester Form enthält.

Die Neutralisation der mit Chlorsulfonsäure oder $SO_3$ umgesetzten Reaktionsmischung sollte unverzüglich nach der Umsetzung erfolgen, um unerwünschte Nebenbeziehungsweise Zersetzungsreaktionen zu vermeiden. Die Neutralisation erfolgt nach an sich bekannten Verfahren in bekannten Einrichtungen, wie sie beispielsweise beschrieben sind in Soap and Chemical Specialities, Vol. 43 (1967), Nr. 5, Seite 124, rechte Spalte und Seite 126, linke Spalte sowie Fig. 3.

Die nach der Neutralisation entstandenen wäßrigen Lösungen des erfindungsgemäß hergestellten Tensidgemisches sind im allgemeinen gebrauchsfertig. Sie können gegebenenfalls, beispielsweise zur Verbesserung ihrer Farbe oder zur weiteren Herabsetzung ihres Gehaltes an 1,4-Dioxan,mit bekannten Verfahren nachbehandelt werden. Die Nachbehandlung zur weiteren Entfernung von Dioxan ist in jedem Fall wesentlich weniger kostenaufwendig als die eingangs als Stand der Technik beschriebenen Verfahren zur Entfernung von 1,4-Dioxan aus Tensidlösungen, die ausschließlich Ethersulfate enthalten.

Die nach dem neuen Verfahren mit Chlorsulfonsäure oder $SO_3$ umzusetzende Mischung der Verbindungen der Formeln (I) und (II) kann entweder durch Zusammenmischen der entsprechenden Verbindungen erzeugt werden oder durch Oxethylierung eines Gemisches von mindestens einem Alkanol und mindestens einem Carbonsäureamid, von dem 1 oder 2 am Stickstoff gebundene Wasserstoffatome durch je eine $-CH_2CH_2OH$-Gruppe substituiert sind.

Gegenstand der Erfindung ist ferner ein in Wasser gelöstes Ethersulfat enthaltendes Tensidgemisch, das enthält: weniger als 0,015 Gew.-%, bezogen auf das Tensidgemisch, 1,4-Dioxan und mehr als 95 Gew.-%, bezogen auf das Tensidgemisch, von einer Ethersulfat-Mischung, die ihrerseits besteht aus:
97 bis 30 Mol-%, bezogen auf die Ethersulfat-Mischung, von mindestens einer Verbindung der Formel

$R-O-(CH_2CH_2O)_aSO_3M$ (III) ,

worin R; a und M die weiter oben beschriebene Bedeutung haben und

3 bis 70 Mol-%, bezogen auf die Ethersulfat-Mischung, von mindestens einer Verbindung der Formeln

$$R^1-CO-N \begin{matrix} \diagup (CH_2CH_2O)_bSO_3M \\ \diagdown (CH_2CH_2O)_dSO_3M \end{matrix} \qquad (IV) \; ,$$

und/oder

$R^1$-CO-NH-$(CH_2CH_2O)_bSO_3M$ (V) ,

in der $R^1$ ; b und M die weiter oben angegebene Bedeutung haben und d eine Zahl von 1 bis 20 bedeutet.

Bevorzugt sind solche Tensidgemische, in denen M in den oben angegebenen Formeln (III), (IV) und (V) bedeutet: Na; K; 1/2 Mg; $NH_4$ oder $NH(CH_2CH_2OH)_3$ .

In einer ebenfalls bevorzugten Ausführungsform bedeutet a in der oben angegebenen Formel (III) eine Zahl von 2 bis 4.

Weiterhin bevorzugt ist ein Tensidgemisch, bei dem $R^1$ in den oben angegebenen Formeln (IV) und (V) ein Alkylrest mit 7 bis 17 C-Atomen ist.

Die neuen Tensidgemische können beispielsweise hergestellt werden nach den weiter oben beschriebenen neuen Verfahren.

Das erfindungsgemäße Verfahren ermöglicht es, in Wasser gelöste, Ethersulfat enthaltende Tensidmischungen herzustellen, die weniger als 0,015 Gew.-%, bezogen auf das Tensidgemisch, 1,4-Dioxan enthalten. Der besondere Vorteil dieses Verfahrens ist, daß die Entstehung von 1,4-Dioxan bereits während der Herstellung des Tensidgemisches in erheblichem Maße vermindert wird, so daß das fertige,in Wasser gelöste Tensidgemisch entweder überhaupt nicht zur Entfernung des restlichen 1,4-Dioxans nachbehandelt werden muß oder wenn extrem niedrige Dioxan-Restgehalte gefordert werden kostengünstig im Vergleich zu den Methoden des Standes des Technik nachbehandelt werden kann. Das neue Verfahren kann in den üblichen Sulfatierungsanlagen mit minimalem apparativen Aufwand durchgeführt werden. Es führt zu Produkten, die beispielsweise als Körperpflegemittel, wie Schaumbäder, Haarwaschmittel oder Kosmetika, ferner als Geschirrspülmittel oder Waschmittel, verwendet werden können.

Nachfolgende Beispiele und Vergleichsversuche sollen die Erfindung näher erläutern:

Beispiele 1 bis 27 und Vergleichsversuche V1 bis V20:

In eine Vorrichtung, bestehend aus einem senkrechtstehenden Rohr und darin konzentrisch angeordnetem zweiten Rohr von kleinerem Durchmesser, werden von oben unter Benetzung der Innenwand des äußeren Rohres und der Außenwand des inneren Rohres je Versuch 1 mol pro Stunde eines der in nachfolgender Tabelle I angegebenen flüssigen Gemisches von Verbindungen der Formel (I) und (II) in verschiedenen Mischungsverhältnissen eingespeist. In den Raum zwischen den beiden konzentrisch angeordneten Rohren wird 1 mol (80 g) pro Stunde Schwefeltrioxid als 2 vol.%ige Mischung mit Stickstoff kontinuierlich eingetragen. Die Außenwand des äußeren Rohres und die Innenwand des inneren Rohres werden mit Wasser von 20 °C gekühlt. Das die Vorrichtung im unteren Teil verlassende Gas enthält 0,01 Vol.-% Schwefeltrioxid. Je 1 mol des Gemisches der Verbindungen der Formeln (I) und (II) werden 0,995 mol Schwefeltrioxid aufgenommen. Durch Anwendung verschiedener Kühlwasser-Strömungsgeschwindigkeiten verlassen die so erhaltenen sulfatierten Gemische die oben beschriebene Vorrichtung mit Temperaturen zwischen 25 und 40 °C, sie werden unmittelbar anschließend eine Stunde lang in eine mit Wasser von 20 °C gekühlte wäßrige Lösung von 39,8 g Natriumhydroxid in verschiedenen Mengen Wasser, die aus nachfolgender Tabelle II ersichtlich sind, eingetragen, wobei die Temperatur des Gemisches nicht über 35 °C ansteigt. Sofern erforderlich, werden nach dem Ende des Eintrags der sulfatierten Mischung der Verbindungen der Formeln (I) und (II) in die wäßrige Natriumhydroxid-Lösung noch geringe Menge einer 20 gew.%igen wäßrigen Natronlauge zugesetzt, um den pH-Wert auf 7 einzustellen. Die Wassermenge, in der das Natriumhydroxid gelöst wird, ist so berechnet, daß nach Eintrag des sulfatierten Gemisches eine wäßrige Lösung der Natriumsalze der Verbindungen der Formeln (III) und (V) vorliegt, die etwa 30 Gew.-% an den genannten Verbindungen enthält. Das Molekulargewicht der in Tabelle I angegebenen Gemische A bis K wird aus den OH-Zahlen der Komponenten des Gemisches die in Tabelle I in Klammern mit der Bezeichnung OHZ jeweils angegeben sind, unter Berücksichtigung des Mischungsverhältnisses der beiden

Komponenten, das für die verschiedenen Versuche aus den Tabellen II und III ersichtlich ist, berechnet.

Von den, wie oben beschrieben, hergestellten 30 gew.%igen wäßrigen Lösungen der Natriumsalze der Verbindungen der Formeln (III) und (V) wird der Dioxangehalt bestimmt. Er ist in Gewichtsprozent, bezogen auf das wasserfreie Gemisch, der Natriumsalze der Verbindungen der Formeln (III) und (V) bei den jeweiligen Versuchen in Tabellen II und III angegeben.

Die Vergleichsversuche V1 bis V10, V21 und V23 werden jeweils nur mit den unvermischten Einzelkomponenten der Formel (I) und die Vergleichsversuche V11 bis V20 sowie V22 und V24 jeweils mit den unvermischten Einzelkomponenten der Formel (II) durchgeführt. Die hierbei ermittelten Dioxanwerte sind aus den Tabellen II und III ersichtlich.

In nachfolgender Tabelle I bedeutet EO Ethylenoxid. Beispielsweise ist "Dodecyl/Tetradecylalkohol + 3 EO" ein Gemisch verschiedener Alkanole mit 12 bis 14 C-Atomen, an das im Durchschnitt 3 Ethylenoxideinheiten angelagert wurden. "Cocosfettsäure" beziehungsweise "Talgfettsäure" sind jeweils Gemische gesättigter aliphatischer Carbonsäuren mit nachfolgend angegebener Kettenlängen-Verteilung (in Gewichtsprozent):

| | $C_8$ | $C_{10}$ | $C_{12}$ | $C_{14}$ | $C_{16}$ | $C_{18}$ |
|---|---|---|---|---|---|---|
| Cocosfettsäure | 7 | 6 | 51 | 19 | 8 | 9 |
| Talgfettsäure | - | - | 1 | 3 | 31 | 65 |

Tabelle I

| Angewendete Gemische von Verbindungen der Formeln (I) und (II) | |
|---|---|
| A) | (I) Dodecylalkohol + 2 EO (OHZ 204) und<br>(II) Cocosfettsäuremonoethanolamid + 1,6 EO (OHZ 203) |
| B) | (I) Dodecyl/Tetradecylalkohol + 3 EO (OHZ 173) und<br>(II) Cocosfettsäuremonoethanolamid + 1,6 EO (OHZ 203) |
| C) | (I) Dodecylalkohol + 2 EO (OHZ 204) und<br>(II) Cocosfettsäuremonoethanolamid (OHZ 224) |
| D) | (I) Dodecyl/Tetradecylalkohol + 3 EO (OHZ 173) und<br>(II) Cocosfettsäuremonoethanolamid (OHZ 224) |
| E) | (I) Dodecylalkohol + 2 EO (OHZ 204) und<br>(II) Cocosfettsäuremonoethanolamid + 5 EO (OHZ 132) |
| F) | (I) Dodecyl/Tetradecylalkohol + 3 EO (OHZ 173) und<br>(II) Cocosfettsäuremonoethanolamid + 5 EO (OHZ 132) |
| G)<br>(II) Talgfettsäuremonoethanolamid + 5 EO (OHZ 146) | (I) Dodecylalkohol + 2 EO (OHZ 204) und |
| H)<br>(II) Essigsäuremonoethanolamid (OHZ 550) | (I) Dodecylalkohol + 2 EO (OHZ 204) und |
| I)<br>(II) Cocosfettsäuremonoethanolamid (OHZ 224) | (I) Nonylphenol + 4 EO (OHZ 155) und |
| K)<br>(II) Cocosfettsäuremonoethanolamid (OHZ 224) | (I) Nonylphenol + 12 EO (OHZ 76) und |

Die bei den Vergleichsversuchen V1 bis V20 und den Beispielen 1 bis 27 angewendeten Molverhältnisse der Verbindungen der Formeln (I) und (II), die Wassermengen und die ermittelten Dioxangehalte der circa 30 gew.%igen wäßrigen Lösungen sind aus nachfolgender Tabelle ersichtlich:

Tabelle II

| | Gemisch der Verbindungen der Formeln (I) und (II) | | | | | | | | | |
|---|---|---|---|---|---|---|---|---|---|---|
| | A | B | C | D | E | F | G | H | I | K |
| Vergleichsversuch | V1 | V2 | V3 | V4 | V5 | V6 | V7 | V8 | V9 | V10 |
| Molverhältnis (I) : (II)*) | 100 : 0 | 100 : 0 | 100 : 0 | 100 : 0 | 100 : 0 | 100 : 0 | 100 : 0 | 100 : 0 | 100 : 0 | 100 : 0 |
| g $H_2O$ ***) | 860 | 970 | 860 | 970 | 860 | 970 | 860 | 860 | 1 060 | 1 930 |
| Dioxangehalt Gew.-% **) | 0,031 | 0,037 | 0,0305 | 0,0385 | 0,032 | 0,039 | 0,047 | 0,0315 | 0,052 | 0,128 |
| Beispiel | 1 | 2 | 3 | 4 | 5 | 6 | - | 7 | 8 | 9 |
| Molverhältnis (I) : (II)*) | 90 : 10 | 88,5 : 11,5 | 89 : 11 | 87,4 : 12,6 | 93,3 : 16,7 | 92,2 : 17,8 | - | 76,9 : 23,1 | 86,2 : 23,8 | 75,3 : 24,7 |
| g $H_2O$ ***) | 860 | 960 | 850 | 955 | 890 | 995 | - | 815 | 1 030 | 1 820 |
| Dioxangehalt Gew.-% **) | 0,0032 | 0,0045 | 0,0037 | 0,0045 | 0,0059 | 0,0071 | - | 0,0036 | 0,0084 | 0,0128 |
| Beispiel | 10 | 11 | 12 | 13 | - | - | - | - | - | - |
| Molverhältnis (I) : (II)*) | 70 : 30 | 66,5 : 33,5 | 68 : 32 | 64,3 : 35,7 | - | - | - | - | - | - |
| g $H_2O$ ***) | 860 | 940 | 840 | 920 | - | - | - | - | - | - |
| Dioxangehalt Gew.-% **) | 0,003 | 0,0034 | 0,0035 | 0,0041 | - | - | - | - | - | - |

(Fortsetzung)

(Fortsetzung)

Gemisch der Verbindungen der Formeln (I) und (II)

| | A | B | C | D | E | F | G | H | I | K |
|---|---|---|---|---|---|---|---|---|---|---|
| Beispiel | 14 | 15 | 16 | 17 | 18 | 19 | 20 | 21 | 22 | 23 |
| Molverhältnis (I) : (II)*) | 50 : 50 | 46 : 54 | 47,7 : 52,3 | 43,6 : 56,4 | 60,7 : 39,3 | 56,7 : 43,3 | 58,3 : 41,7 | 27 : 73 | 40,9 : 59,1 | 25,3 : 74,7 |
| g $H_2O$ ***) | 860 | 915 | 830 | 885 | 1 030 | 1 090 | 985 | 655 | 930 | 1 370 |
| Dioxangehalt Gew.-% **) | 0,003 | 0,0036 | 0,0032 | 0,0033 | 0,0056 | 0,0063 | 0,0092 | 0,0031 | 0,0076 | 0,0112 |
| Beispiel | 24 | 25 | 26 | 27 | - | - | - | - | - | - |
| Molverhältnis (I) : (II)*) | 30 : 70 | 26,7 : 73,3 | 28 : 72 | 24,9 : 75,1 | - | - | - | - | - | - |
| g $H_2O$ ***) | 860 | 895 | 820 | 850 | - | - | - | - | - | - |
| Dioxangehalt Gew.-% **) | 0,0031 | 0,0035 | 0,0029 | 0,0034 | - | - | - | - | - | - |
| Vergleichsversuch | V11 | V12 | V13 | V14 | V15 | V16 | V17 | V18 | V19 | V20 |
| Molverhältnis (I) : (II)*) | 0 : 100 | 0 : 100 | 0 : 100 | 0 : 100 | 0 : 100 | 0 : 100 | 0 : 100 | 0 : 100 | 0 : 100 | 0 : 100 |
| g $H_2O$ ***) | 860 | 860 | 800 | 800 | 1 205 | 1 205 | 1 130 | 455 | 800 | 800 |
| Dioxangehalt Gew.-% **) | 0,003 | 0,0028 | 0 | 0 | 0,0051 | 0,0052 | 0,0086 | 0 | 0 | 0 |

Fußnoten zur Tabelle II:

*) Molverhältnis in Mol-% der Verbindungen der Formel (I) zu den Verbindungen der Formel (II). Mittlere Molgewichte aus den OH-Zahlen berechnet.

**) bezogen auf das wasserfreie Gemisch der Verbindungen der Formeln (III) und (V)

***) Wassermenge, in der die 39,8 g NaOH gelöst werden

Vergleichsversuche V21 und V22 sowie Beispiele 28 bis 30:

Es wird verfahren wie bei den Vergleichsversuchen V1 bis V20 und Beispielen 1 bis 27 angegeben, jedoch wird anstelle der 2 Vol.-% Schwefeltrioxid enthaltenden Gasmischung eine 7 Vol.-% Schwefeltrioxid enthaltende Mischung verwendet, wobei wiederum 1 mol (80 g) pro Stunde Schwefeltrioxid in die Vorrichtung eingetragen werden. Das Restgas, das die Vorrichtung verläßt, enthält 0,03 Vol.-% Schwefeltrioxid, demnach wurden 0,996 mol Schwefeltrioxid von dem flüssigen Gemisch der Verbindungen der Formeln (I) und (II) aufgenommen. Das angewendete Verbindungsgemisch entspricht dem unter A) in Tabelle I angegebenen. Für die Vergleichsversuche V21 beziehungsweise V22 werden die reinen Einzelkomponenten des Gemisches eingesetzt. Das Molverhältnis der Verbindungen der Formel (I) und (II), die eingesetzte Wassermenge und der an den erzeugten circa 30 Gew.-% der Natriumsalze der Verbindungen der Formeln (III) und (V) enthaltenden wäßrigen Lösungen ermittelte Dioxangehalt ist aus nachfolgender Tabelle III ersichtlich.

Vergleichsversuche V23 und V24 sowie Beispiele 31 bis33:

Es werden für die Vergleichsversuche die reinen Komponenten, für die Beispiele die Mischung der Verbindungen der Formeln (I) und (II), die in Tabelle I unter C) aufgeführt ist, verwendet. In einem 2 $dm^3$ fassenden Rührkolben, der ein Thermometer enthält und von außen mit Wasser kühlbar ist, werden für jeden Versuch 2 mol der oben näher beschriebenen Verbindungen und Gemische in verschiedenen Mischungsverhältnissen der Einzelkomponenten vorgelegt und unter Rühren und Kühlen mit Wasser von 20 °C 2 mol (233 g) Chlorsulfonsäure unter Aufrechterhaltung einer Innentemperatur von 30 °C zugetropft und anschließend der Rührkolben auf einen Druck von 2,6 kPa evakuiert, um den Rest des entstandenen Chlorwasserstoffgases zu entfernen. Die so erhaltenen sulfatierten Reaktionsgemische werden in eine wäßrige Lösung eingetragen, die 79,5 g Natriumhydroxid und die jeweils in Tabelle III angegebenen Mengen Wasser enthält, wobei durch Kühlung dafür gesorgt wird, daß während der Reaktion des sulfatierten Gemisches mit der Natriumhydroxidlösung die Temperatur nicht über 35 °C ansteigt. Nach Beendigung des Eintrags wird, falls erforderlich, durch Zugabe kleinerer Mengen einer 20 Gew.-% Natriumhydroxid enthaltenden wäßrigen Lösung, der pH-Wert auf 7 eingestellt. Von den so erzeugten, etwa 30 Gew.-% der Natriumsalze der Verbindungen der Formeln (III) und (V) enthaltenden wäßrigen Lösungen, wird der Dioxangehalt bestimmt. Er ist in nachfolgender Tabelle III in Gew.-%, bezogen auf wasserfreie Natriumsalze,der Verbindungen (III) und (V) angegeben.

Tabelle III

| | 1 mol $SO_3$ in 7 vol.%iger Gasmischung<br>Gemisch der Verbindungen (I) und (II): A | 2 mol Chlorsulfonsäure<br>Gemisch der Verbindungen (I) und (II): C |
|---|---|---|
| Vergleichsversuch | V21 | V23 |
| Molverhältnis (I) : (II)*) | 100 : 0 | 100 : 0 |
| g $H_2O$ ***) | 860 | 1 720 |
| Dioxangehalt Gew.-% **) | 0,038 | 0,024 |
| Beispiel | 28 | 31 |
| Molverhältnis (I) : (II)*) | 90 : 10 | 89 : 11 |
| g $H_2O$ ***) | 860 | 1 700 |
| Dioxangehalt Gew.-% **) | 0,0048 | 0,0028 |
| Beispiel | 29 | 32 |
| Molverhältnis (I) : (II)*) | 50 : 50 | 47,7 : 52,3 |
| g $H_2O$ ***) | 860 | 1 660 |
| Dioxangehalt Gew.-% **) | 0,0041 | 0,0022 |
| Beispiel | 30 | 33 |
| Molverhältnis (I) : (II)*) | 30 : 70 | 28 : 72 |
| g $H_2O$ ***) | 860 | 1 640 |
| Dioxangehalt Gew.-% **) | 0,0037 | 0,0023 |

(Fortsetzung)

(Fortsetzung)

| | 1 mol $SO_3$ in 7 vol.%iger Gasmischung<br>Gemisch der Verbindungen (I) und (II): A | 2 mol Chlorsulfonsäure<br>Gemisch der Verbindungen (I) und (II): C |
|---|---|---|
| Vergleichsversuch | V22 | V24 |
| Molverhältnis (I) : (II)*) | 0 : 100 | 0 : 100 |
| g $H_2O$ ***) | 860 | 1 600 |
| Dioxangehalt Gew.-% **) | 0,0035 | 0,0021 |

Fußnoten zur Tabelle III:

wie die in Tabelle II

## Ansprüche

1. Verfahren zur Herstellung eines Ethersulfat enthaltenden Tensidgemisches mit niedrigem Dioxangehalt, dadurch gekennzeichnet, daß eine Mischung, bestehend aus 99 bis 5 Mol-%, bezogen auf die Mischung, von mindestens einer Verbindung der Formel

$R\text{-}O\text{-}(CH_2CH_2O)_aH$ (I),

worin bedeuten
R einen Alkylrest mit 8 bis 18 C-Atomen oder einen Alkylphenylrest, der mit 1 bis 3 Alkylresten substituiert ist, wobei die Gesamtzahl der C-Atome in den Alkylresten 4 bis 12 beträgt,
a eine Zahl von 1 bis 10 und
1 bis 95 Mol-%, bezogen auf die Mischung, von mindestens einer Verbindung der Formel

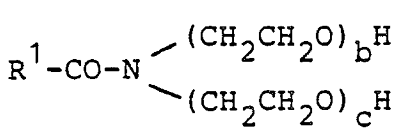 (II) ,

worin bedeuten
$R^1$ einen Alkylrest mit 1 bis 17 C-Atomen,
b eine Zahl von 1 bis 20 und
c Null oder eine Zahl von 1 bis 20

in flüssigem Zustand bei 20 bis 120 °C mit 0,95 bis 1 mol Chlorsulfonsäure oder $SO_3$ je 1 mol der in den Verbindungen der Formeln (I) und (II) enthaltenen $-CH_2CH_2OH$-Gruppen umgesetzt werden und das Reaktionsprodukt anschließend mit einer wäßrigen Lösung oder Aufschlämmung einer Verbindung der Formel MOH, worin bedeutet M = Na; K; Li; 1/2 Mg; 1/2 Ca; 1/2 Ba; oder

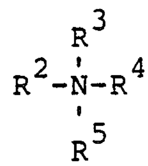 ,

worin $R^2$ bis $R^5$ gleich oder verschieden sein können und jedes für sich bedeutet: H; Alkyl mit 1 bis 4 C-Atomen oder $-CH_2CH_2OH$, neutralisiert wird.

2. Verfahren nach Anspruch 1, dadurch gekennzeichnet, daß 97 bis 30 Mol-%, bezogen auf die Mischung, der Verbindung der Formel (I) und 3 bis 70 Mol-%, bezogen auf die Mischung, der Verbindung der Formel (II) mit Chlorsulfonsäure oder $SO_3$ umgesetzt werden.

3. Verfahren nach Anspruch 1 oder 2, dadurch gekennzeichnet, daß die Mischung der Verbindungen der Formeln (I) und (II) mit einer Gasmischung, die neben nicht reagierenden Gasen 0,5 bis 10 Vol.-%, bezogen auf die Gasmischung, $SO_3$ enthält, solange in Kontakt gebracht wird, bis je 1 mol der in den Verbindungen (I) und (II) enthaltenen-$CH_2CH_2OH$-Gruppe 0,95 bis 1 mol $SO_3$ von der Mischung der Verbindungen der Formeln (I) und (II) aufgenommen worden sind.

4. Verfahren nach einem oder mehreren der Ansprüche 1 bis 3, dadurch gekennzeichnet, daß R in Formel (I) ein Alkylrest mit 10 bis 16 C-Atomen ist.

5. Verfahren nach einem oder mehreren der Ansprüche 1 bis 4, dadurch gekennzeichnet, daß a in Formel (I) eine Zahl von 2 bis 4 ist.

6. Verfahren nach einem oder mehreren der Ansprüche 1 bis 5, dadurch gekennzeichnet, daß b in Formel (II) eine Zahl von 1 bis 6 und c gleich Null ist.

7. Verfahren nach einem oder mehreren der Ansprüche 1 bis 6, dadurch gekennzeichnet, daß $R^1$ in Formel (II) ein Alkylrest mit 7 bis 17 C-Atomen ist.

8. Verfahren nach einem oder mehreren der Ansprüche 1 bis 7, dadurch gekennzeichnet, daß M = Na; K; 1/2 Mg; $NH_4$ oder $NH(CH_2CH_2OH)_3$ ist.

9. Verfahren nach einem oder mehreren der Ansprüche 3 bis 8, dadurch gekennzeichnet, daß die Gasmischung 1 bis 5 Vol-% $SO_3$ enthält.

10. In Wasser gelöstes Ethersulfat enthaltendes Tensidgemisch, das enthält: weniger als 0,015 Gew.-%, bezogen auf das Tensidgemisch, 1,4-Dioxan und mehr als 95 Gew.-%, bezogen auf das Tensidgemisch, von einer Ethersulfat-Mischung, die ihrerseits besteht aus:
97 bis 30 Mol-%, bezogen auf die Ethersulfat-Mischung, von mindestens einer Verbindung der Formel

$R-O-(CH_2CH_2O)_aSO_3M$ (III) ,

worin R; a und M die in Anspruch 1 genannte Bedeutung haben und
3 bis 70 Mol-%, bezogen auf die Ethersulfat-Mischung von mindestens einer Verbindung der Formeln

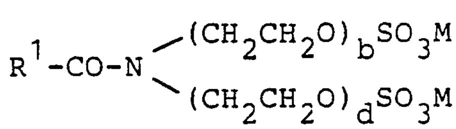 (IV)

und/oder

$R^1-CO-NH(CH_2CH_2O)_bSO_3M$ (V),

in der $R^1$; b und M die in Anspruch 1 angegebene Bedeutung haben und d eine Zahl von 1 bis 20 bedeutet.

11. Tensidgemisch nach Anspruch 10, dadurch gekennzeichnet, daß M bedeutet: Na; K; 1/2 Mg; $NH_4$ oder $NH(CH_2CH_2OH)_3$ .

12. Tensidgemisch nach Anspruch 10 oder 11, dadurch gekennzeichnet, daß a in Formel (III) eine Zahl von 2 bis 4 ist.

13. Tensidgemisch nach einem oder mehreren der Ansprüche 10 bis 12, dadurch gekennzeichnet, daß $R^1$ in Formel (IV) und (V) ein Alkylrest mit 7 bis 17 C-Atomen ist.

Europäisches Patentamt

# EUROPÄISCHER RECHERCHENBERICHT

Nummer der Anmeldung

EP 88 12 0955

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-1 815 657 (FARBWERKE HOECHST AG)<br>* Gesamtes Dokument *<br>--- | 1-13 | C 11 D 11/04<br>C 07 C 141/04 |
| Y | FR-A-1 293 265 (SIPON PRODUCTS LIMITED)<br>* Gesamtes Dokument *<br>--- | 1 | |
| Y | ATLAS POWDER CORPORATION<br>DE - A - 20779 IVb * Anspruch; Seite 2, Zeilen 36-39 *<br>----- | 1 | |

RECHERCHIERTE SACHGEBIETE (Int. Cl.4)

C 11 D
C 07 C

Der vorliegende Recherchenbericht wurde für alle Patentansprüche erstellt

| Recherchenort | Abschlußdatum der Recherche | Prüfer |
|---|---|---|
| BERLIN | 17-02-1989 | PELLI-WABLAT B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)